Europäisches Patentamt

**European Patent Office**

Office européen des brevets

(19)

(11) Publication number: **0 061 250**
**A2**

(12) # EUROPEAN PATENT APPLICATION

(21) Application number: **82301163.0**

(22) Date of filing: **08.03.82**

(51) Int. Cl.³: **C 12 P 1/00**
**C 12 N 1/06, C 12 P 21/00**
**//C12N15/00**

(30) Priority: **23.03.81 GB 8108982**

(43) Date of publication of application:
. **29.09.82 Bulletin 82/39**

(84) Designated Contracting States:
**AT BE CH DE FR GB IT LI LU NL SE**

(71) Applicant: **BIOGEN N.V.**
**15 Pietermaai**
**Willemstad Curacao, Netherlands Antilles(NL)**

(72) Inventor: **Weissman, Charles**
**Eschenhausstrasse 34**
**CH-8053 Zurich(CH)**

(72) Inventor: **Schein, Catherine**
**Ekkehardstrasse 32**
**CH-8006 Zurich(CH)**

(74) Representative: **Hartley, David et al,**
**c/o Withers & Rogers 4 Dyer's Buildings Holborn**
**London, EC1N 2AJT(GB)**

(54) Improved process for recovering products produced by host organisms.

㊲ An improved process for recovering a product from a host organism that produces such product. The process is charaterized by the step of exposing a host organism that produces a product to at least one composition which causes lysis or at least permeabilization of the host organism and release of the desired product into the culture medium in recoverable form.

EP 0 061 250 A2

## IMPROVED PROCESS FOR RECOVERING
## PRODUCTS PRODUCED BY HOST ORGANISMS

### TECHNICAL FIELD OF INVENTION

This invention relates to methods for producing proteins, polypeptides or other products in host organisms. More particularly, it relates to a method for recovering a desired protein, polypeptide, or other product from a host organism which produces that product. The method of this invention is characterized by the step of exposing the host organism to at least one composition which causes lysis or at least permeabilization of the host and release of the desired product into the culture medium in recoverable form. As will be appreciated from the disclosure to follow, the method of this invention is generally useful in the production of various proteins, polypeptides and other products in various host organisms.

### BACKGROUND ART

One of the problems that besets the large-scale production of proteins, polypeptides and other products in potentially hazardous host organisms is how to inactivate or kill the host organisms of the culture while at the same time being able to recover the desired product.

This problem has become even more important because of the development of recombinant DNA technology. Such technology permits the production of foreign products in host organisms that have been transformed with foreign DNA sequences that code for those products. Examples of such processes include the production of human insulin, human interferons, hepatitis B viral antigens, foot and mouth disease viral antigens, and human growth hormone in bacteria and other host organisms.

Two general approaches to this problem have been employed in the prior art. In the first of these, the host organisms of the growing culture are inactivated under appropriate conditions of physical containment, by heat, acid or other inactivating treatment before harvesting. Then, the inactivated host organisms are harvested and the desired proteins or polypeptides are recovered. In the second of these prior approaches, the host organisms are harvested and extracted under conditions of physical containment, as required by the appropriate governmental guidelines and regulations. Then, any live cells remaining are inactivated or killed as before and the desired proteins or polypeptides are recovered.

However, neither of these approaches has proven satisfactory in the recovery of desired products from large-volume fermentations of host organisms which produce those products. For example, in the case of the microbial production of human leukocyte interferon (IFN-α) from E.coli transformed with a DNA sequence that codes for IFN-α (such microorganisms being, for example, described in European patent application 81.300050.2, filed January 7, 1981, which application is incorporated herein by reference) the first general approach -- inactivating the transformed host organisms by either heat, acid or other inactivating treatment followed by harvesting -- gave a yield of not more than about 10% of the interferon or interferon-like products produced by the microorganisms. While not wishing to be bound by theory, it is believed that the other products of the host organism complex with or occlude the desired interferon product and prevent its recovery from the inactivated and harvested culture.

The second general approach, while perhaps usable on a laboratory scale, is technically difficult to implement on a larger scale. For example, the complexity and expense of fermentation and processing equipment to provide the required containment during harvesting and extraction of large-scale cultures of living cells is high. Moreover, the procedures which must be employed are bulky and not preferred, both from an equipment and personnel standpoint.

## DISCLOSURE OF THE INVENTION

The present invention solves the problems referred to by providing a method for recovering a desired protein, polypeptide or other product from a host organism which produces that product.

By virtue of this invention, it is possible to release into the culture medium in recoverable form substantially all of the desired protein, polypeptide or other product produced by the host organism, while at the same time inactivating the host organism.

The improved process of this invention is therefore distinguishable from the prior processes, above-described, in that this process affords substantially the complete recovery of the desired products from a culture of host organisms and at the same time avoids the disadvantages of the prior art processes.

## BEST MODE OF CARRYING OUT THE INVENTION

In order that the invention herein described may be more fully understood, the following detailed description is set forth. In this description the term "host organism" is used broadly. For example, it includes both gram negative and gram positive bacterial hosts such as strains of E.coli, e.g., E.coli HB101, E.coli X1776, E.coli X2882, E.coli DS410, E.coli MRCI, and strains of Pseudomonas, Bacillus subtilis, Bacillus steareothermophilus and other bacilli, yeasts, and other fungi, animal or plant hosts such as animal (including human) or plant cells in culture or other hosts. The selection of a particular host is not part of this invention. Rather, the invention is applicable generally to the recovery of products from all hosts that are lysable or at least permeablizable on exposure to the compositions of this invention.

In addition, the process of this invention is applicable to a wide variety of proteins, polypeptides and other products. For example, the recovery of products such as the active components of vaccines or other pharmaceutically or internally useful compositions, enzymes, alkaloids, amino acids, industrial chemicals, antibiotics, food stuffs, and the like are part of this invention. Again, the selection of a particular product is not part of this invention. Rather, the inventin is applicable generally to the recovery of all such desired products.

Finally, the numerous techniques and processes for growing or fermenting cultures of host organisms or the purification of products from them are not part of this invention. Rather, this invention is a useful complement to those techniques and enables the improved recovery of the desired product from them.

It should, of course, be understood that not every host or every growth or parification technique may be usefully employed to produce a specific product in a particular host organism by the processes of this invention Rather, those of skill in the art should consider various factors known in the art to select a preferred host and preferred conditions for the production and purification of a desired product.

For example, a wide variety of host/cloning vehicle combinations have been employed in recombinant DNA technology to prepare transformed host organisms that produce a desired foreign product. For example, useful cloning vehicles may consist of segments of chromosomal, non-chromosomal and synthetic DNA sequences, such as various known bacterial plasmids, e.g., plasmids from E.coli including col El, pCRl, pBR322 and their derivatives wider host range plasmids, e.g., RP4; phage DNA, e.g., the numerous derivatives of phage λ, e.g., NM989; and vectors derived from combinations of plasmids and phage DNAs such as plasmids which have been modified to employ phage DNA or other expression control sequences or yeast plasmids such as 2μ plasmids or derivatives thereof. Various DNA sequences may be put into those cloning treatment. And, yeast cells are more susceptible to Glusulase®.

·The various compositions, described above as illustrative of the compositions useful in the process of this invention, have a well-known spectrum of activities against various host organisms. Therefore, the choice of a composition in accordance with this invention for a particular application must be governed by a number of factors. These include the susceptibility of the host organism to the composition, the susceptibility of the host organism after transformation, if any, with a hybrid plasmid to the composition, the activity and stability of the composition in the culture medium, the stability of the desired product in the culture medium and in subsequent processing steps and any interaction between the composition and the desired product. Using these principles, one of skill in the art may choose a particular composition within the scope of this invention and usefully employ · the process of this invention in the recovery of a desired product.

The "schedule" for exposing the host organism to the chosen composition in the process of this invention is also governed to some extent by the particular host organism, composition employed and the products desired. For example, some treatments like lysozyme/EDTA followed by exposure to SDS require that the growth of the culture of host organisms be substantially complete before beginning the release of the product. On the other hand, exposure to penicillin or its derivatives, such as ampicillin or other like acting compositions, permits a wide variety of schedules to be followed. For example, the amount of the composition added, the time in the host organism growth· cycle of the addition, the number of additives, the number of additions or the period of host organism growth after addition and before product recovery are all subject to modification by those of skill in the art without departing from the scope of this invention.

vehicles at well-recognized sites in them to produce hybrid DNA molecules. These molecules are useful, either as produced or after appropriate modification to improve their expression characteristics or to modify the product actually expressed from them, to transform host organisms, as described above, and to produce the products coded for by the inserted DNA sequences or desired fragments thereof.

Illustrative of the foreign products that have been produced by such transformed host organisms are hepatitis B viral antigens, foot and mouth disease viral antigens, leukocyte interferon, fibroblast interferon, the A and B chains of human insulin, rat growth hormone, mouse dihydrofolate reductase, somatostatin, human growth hormone and the like.

Within the above general technology, the present invention is characterized by the step of exposing a host organism to at least one composition to which the host organism responds by lysing or at least becoming permeable so as to release the desired product made by it into the culture medium. These compositions fall into two general classes. The first class dissolves the lipids of the cell membrane and destroys the impermeability of the host organism. Examples of this class are detergents such as triton and sodium dodecyl sulfate ("SDS") and enzymes such as Glusulase® (a β-glucuronidase sulfatase preparation) The second class disrupts the cell envelope of the host organism or blocks enzymes required by the cell wall system. Examples of this class are penicillin, cycloserine, bacitracin, zancomycin, ristocetin, polymyxin and their derivatives, synthetic, semi-synthetic or natural.

It is of course, to be understood that certain host organisms are more susceptible to one class or member of a class of compositions than others. For example, human cells are readily susceptible to nonionic and ionic detergents. On the other hand, E. coli cells are more susceptible to exposure to penicillin or its derivatives or to exposure to SDS following a lysozyme/EDTA

In a batch process, a portion of the culture may be removed at intervals and treated as above to recover the desired product while the remaining culture continues to grow and to produce the desired product. In a continuous process, the desired product may be recovered as above from the medium stream after it has passed through the immobilized or otherwise located culture of host organisms.

In order that this invention may be better understood, the following examples are set forth. These examples are for purposes of illustration only and this invention should not be considered to be limited by any recitation used therein.

## Example I

E.coli DS410 (β-Lac-AUG(α2)), an E.coli DS410 (ara azide Ton A lac y Tsx min a min b gal λ xyl step$^R$) strain, was transformed with plasmid β-lac-AUG(α2) as described in the above-identified European patent application. It is deposited in the American Type Culture Collector under ATCC No. 31769. The transformed host contains the IFN-α2 gene connected via an AUG initiator codon to the penicillinase (β-lac) expression control sequence of pBR322. The host is resistant to tetracycline and streptomycin. It was grown to a cell density of $5 \times 10^9$ ($OD_{650} = 6$) in a 120 l culture at 30°C and a sample arrayed to determine how much IFW had been produced by the host organisms. The culture medium consisted of casamino acids (enzymatic casein hydrolysate C-0626, Sigma) (30 g/l), glycerol (98% PHHVI, pure, Siegfried) (20 g/l), yeast extract (BBL) (2 g/l), $KH_2PO_4$ (pure, p.A. Fluka) (5 g/l), NaOH (p.A. Merck, No. 6498) (1.2 g/l), Silicone DX31DB (Dow Chemical) (0.1 ml/l), $MgSO_4 \cdot 7H_2O$ (Merck, z.Analyse, No. 5886) (1 g/l) and NaOH (as above) (ca 2 g/l).* Aeration and agitation were carried out to

---

* The medium was prepared by autoclaving the casamino acids, glycerol, yeast extract, $KH_2PO_4$ and silicone for 20 min at 120°C. After cooling the $MgSO_4 \cdot 7H_2O$ in 1 ml $H_2O$, which had been separately autoclaved as above, was added and the pH adjusted to 7 with NaOH in $H_2O$.

For example, in one embodiment of this invention a single large dose of penicillin was added to a growing microorganism culture at the end of its growth period. Any host organisms remaining after the time selected for exposure were then inactivated and the desired product recovered. In a second example, the concentration of the penicillin in the culture was kept at a low, more or less, constant level to permit the microorganisms to continue to grow and to produce the desired product at about the same rate that microorganisms were being lysed or at least permeablized by the penicillin to release the desired product. Therefore, proper choice of addition schedules may allow an approximate steady state condition to be approached in the culture. Such procedures are plainly useful in semi-continuous and continuous fermentation and recovery processes.

While not wishing to be bound by theory, it is believed that the process of this invention causes lysis or at least permeabilization of a portion or all of the host organisms of the growing culture and the release of the desired products made by those host organisms into the culture medium. The non-lysed portion of the culture, if any, of course may continue to grow and to produce the desired product until it is lysed or permeabilized by the process of this invention or otherwise inactivated.

In a non-continuous process, the fermentation is ended at some time, preferably by the addition of a sufficient amount of the composition of this invention. Then, any host organisms remaining in an unlysed state are killed by acid or heat treatment, whichever is more favorable to the stability of the desired product. The product is then isolated and purified in an appropriate fashion. For example, acid-stable, acid-precipitable products (e.g. IFN-α, IFN-β) are purified by precipitation with acid and recovered by extraction. Other products are recovered from the cleared lysate by absorption to ion exchange resins or affinity resins or by precipitation with any of a variety of well-known additives, such as salts, metal ions and the like.

ensure not less than 80% dissolved oxygen (relative to air at atmospheric pressure) at any time. The pH was kept to 7 by addition of 10M NaOH.

75 mg/l of ampicillin (a penicillin derivative) were added at $OD_{656} = 6$ and aeration and agitation continued as before. After the $OD_{656}$ decreased to 4 (30-60 min), the culture was then cooled to 10°C and $H_2SO_4$ (ca 4 M) was added slowly to adjust the pH to 1.9. In some instances trichloroacetic acid was employed to adjust the pH to 1.3 so as to favor complete precipitation of the desired IFN product.

The precipitate was collected by a Westphalia separator and the concentrated solids centrifuged to a paste in a Sharples. The paste (about 133g/l, moist) was suspended in 5X its weight of deionized water, homogenized with a Silverson homogenizer and the pH adjusted to 7.2 with 2 M NaOH (about 3l of NaOH are usually required). An assay revealed that substantially all of the IFN produced by the host organism had been recovered.

This harvested and extracted IFN-α may then be treated in a variety of ways to purify it. As above stated, these subsequent techniques are not part of this invention.

## Example II

E.coli DS410 (β-lac AUG(α2)), described above, was grown to a cell density of $10^9$/ml under the same conditions and using the same culture medium as described above. Again, a sample was assayed to determine the level of IFN production. Fifteen μg of ampicillin per ml of culture were then added and incubation continued under full aeration and agitation. No increase in living cells was noted so presumably the fraction of the cells being lysed or at least permeabilized by the ampicillin and releasing IFN-α2 into the medium was being replaced, at least insofar as the limit of detection used, by the remaining cells continued division. These cells, of course, continue to produce additional IFN-α2.

After about 1 hour, an assay showed as high a titer as $200 \times 10^6$ units of IFN/1. The culture was then treated as described in example I and substantially all of the IFN activity recovered from the culture in the acid precipitate.

## Example III

A 1 l culture of E.coli DS410 (β-Lac AUG (α2), described above, was grown at 37°C in the culture medium described above to a cell density of about $10^{10}$. The culture was then incubated with 20 mM EDTA, 100 µg/ml lysozyme for 30 min at 37°C and then lysed with 0.4% SDS. After cooling to 10°C, the lysate was adjusted to pH 2 with sulfuric acid and allowed to stand for 60 min so as to kill all of the remaining cells. The precipitate was collected by centrifugation and homogenized with water (1/20 original culture volume), while adjusting the pH to 7.3 with NaOH. Removal of the insoluble matter gave a clear solution having 50-100% of the original IFN-α activity.

In this instance, further purification of the IFN-α was difficult presumably because of IFN-complexing with the hydrophobic membrane proteins also extracted by the SDS. Such complexing, of course, should not be a disadvantage in the recovery of other non-interferon products.

While we have hereinbefore presented a number of embodiments of this invention, it is apparent that our basic construction can be altered to provide other embodiments which utilize the processes and compositions of this invention. Therefore, it will be appreciated that the scope of this invention is to be defined by the claims appended hereto rather than the specific embodiments which have been presented hereinbefore by way of example.

Claims:

1.  A process for recovering a product from a host organism that produces said product characterized by the step of exposing the host organism to at least one composition which causes lysis on at least permeablization of the host organism and release of the desired product.

2.  The process of claim 1 characterized in that the host organism is selected from the group consisting of strains of E.coli, Pseudomonas, bacillus subtilis, bacillus stearothermophilus, other bacilli, yeasts, other fungi, animal or plant cells and other host organisms.

3.  The process of claim 1 or 2 characterized in that the host organism is a strain of E.coli.

4.  The process of any one of claims 1 to 3 characterized in that the host organism is a host organism that has been transformed with a vector comprising a DNA sequence that codes for said product.

5.  The process of claim 4 characterized in that said DNA sequence is selected from the group consisting of DNA sequences that code for proteins, DNA sequences that code for polypeptides, DNA sequences that code for single or multiple amino acids, DNA sequences that code for enzymes, DNA sequences that code for industrial chemicals and DNA sequences that code for food stuffs.

6.  The process of claim 4 or 5 characterized in that said DNA sequence is selected from the group consisting of DNA sequences that code for polypeptides displaying an immunological or biological activity of leukocyte interferon, DNA sequences that code for polypeptides displaying an immunological or biological activity of fibroblast interferon, DNA sequences that code for polypeptides having the antigenicity of FMDV viral antigens, DNA sequences that code for polypeptides having the antigenicity of hepatitis B viral antigens, and DNA sequences that code for other polypeptides and proteins displaying an immunological or biological activity of animal or human products.

7. The process of any one of claims 4 to 6 characterized that said DNA sequence codes for polypeptide displaying an immunological or biological activity of leukocyte interferon.

8. The process of any one of claims 1 to 7 characterized in that said composition is selected from the group consisting of detergents, enzymes, penicillin, cycloserine, bacitracin, zancomycin, ristocetin, polymyxin and their derivatives.

9. The process of claim 8 characterized in that said composition is selected from the group consisting of penicillin and derivatives thereof.

10. The process of claim 9 wherein said penicillin is added to the culture of about $10^9$ cells/ml in an amount from about 15 $\mu$g/l to about 75 mg/l.